# EUROPEAN PATENT APPLICATION

(11) **EP 4 275 680 A1**
(43) Date of publication of application: **15.11.2023**
(21) Application number: 22172901.5
(22) Date of filing: 12.05.2022
(51) Int. Cl.: A61K 31/198, A61P 11/00

(54) **COMPOSITION COMPRISING CREATINE FOR USE IN THE TREATMENT OF BREATHING DIFFICULTIES**

(71) Applicant: AlzChem Trostberg GmbH, 83308 Trostberg (DE)
(72) Inventor: Bezler, Jürgen, 84489 Burghausen (DE); Löber, Verena, 83376 Truchtlaching (DE); Ostojic, Sergej M., 26000 Pancevo (RS); ALBER, Robert, 83209 Prien (DE)

(57) **Abstract**

The present invention relates to a pharmaceutical composition comprising creatine or physiologically acceptable derivatives and/or salts and or adducts thereof for use in the treatment of breathing difficulties, chest pain, body aches etc. after virus infection particularly in combination with breathing exercises. A further embodiment of the invention relates to the use of a composition comprising creatine or physiologically acceptable derivatives and/or salts and or adducts thereof as dietary supplement for preparation of a diet supporting the recovery from breathing difficulties, chest pain, body aches etc. by breathing exercises after a viral infection of the lungs or lower respiratory system.

## Description

### Field of the invention:

The present invention is related to pharmaceutical compositions comprising creatine for use in the treatment of symptoms caused by virus infection of the lungs or the lower respiratory system, in particular breathing difficulties and chest pain, in a subject in need thereof. A further object of the invention is the use of creatine or creatine derivatives as dietary supplement for the preparation of a diet for patients suffering under breathing difficulties and chest or lung pain.

### Description of the prior art:

Although COVID-19 is seen as a disease that primarily affects the lungs, it can also damage other organs, including the heart, vascular system, kidneys and brain for example. Organ damage increases the risk of sequelae, such as cognitive impairment, heart complications (myocarditis), chronic kidney impairment, stroke, thrombosis, and Guillain-Barre syndrome.

However, most people who are infected with SARS-CoV-2 recover within a few weeks. But some people, even those who had mild versions of the disease, continue to experience a brought variety of symptoms after their initial recovery.

Typical symptoms that may persist after a SARS-CoV-2 infection are for example fatigue (e.g. post-viral fatigue syndrome (PVFS) or chronic fatigue syndrome (CFS)), breathing difficulties or breathlessness (dyspnea), lung or chest pain, joint pain, muscle pain or headache, concentration and memory difficulties, sleep problems (insomnia), loss of smell (anosmia) or taste (ageusia) etc., wherein fatigue, breathing difficulties (dyspnea) and chest pain belongs to the most reported conditions after acute SARS-CoV-2 infection. These symptoms sometimes persist for months in many cases.

In serious courses of disease, more than 50% of COVID-patients suffer from difficulties in breathing after 8 weeks after their discharge from the hospital as reported in a study by Manal S., Barnett J., Brill S. et al. (Thorax, 2021; 76, 396-398). These findings have been confirmed by a survey by Halpin S.J., Mclvor C., et al. (J. Med. Virol. 2021; 93, 1013-1022). More than 60% patients, who received intense medical treatment, suffered from breathing difficulties after in average 48 days after their discharge from the hospital.

The described conditions are summarized under the term "Post-COVID-19 syndrome" or "Long-COVID-19", hereinafter referred to as Post-COVID.

Subjects with breathing difficulties often get not enough air or feeling out of breath. They need to rest frequently during activity or feeling like the upper body and muscles are working harder than normal to breathe. Recommendations for these subjects are usually limited to behavioral advices, such as to allow regular rest periods, to break large tasks in smaller ones and to increase exercise slowly. The situation is very exhausting for subjects suffering under these symptoms.

People with Post-COVID often further experience muscle pain, joint pain (body pain) or chest / lung pain. Unfortunately, until to date, no specific treatments have been found for the cure of this virus. Moscatelli, F. et al., (Nutrients 2021; 13, 976-988) discuss the role of nutritional inventions and highlight the fact that strong data from clinical trials are needed to support any such assumption. Adequate nutrition is required to support the immune cell function by allowing to engage robust responses to pathogens. The micronutrients with the strongest evidence for immune support are vitamins C, D and zinc.

Practical medical treatment strategies of patients suffering from Long-COVID are summarized in Deutsches Ärzteblatt 2020; 49, 117 and are based on primary care recommendations of Long-Covid as described by Greenhalgh, T.; Knight M., A'Court C.; BMJ 2020; 370, 3026. Medical management is limited to symptomatic, such as treating fever with paracetamol and considering antibiotics for secondary infection.

To improve the recovery of patients with breathing difficulties or lung pain, pulmonary rehabilitation treatment should be applied as soon as possible. Pulmonary rehabilitation is regarded as one of the most important interventions in the treatment of Post-COVID, so far.

Suitable pulmonary rehabilitation measures are described in Wang T. J. et al. (Am. J. Phys. Med. Rehabil., 2020 Sep; 99(9), 769-774).

Creatine is a methylguanidinoacetic acid usually available from animal-based foods and/or produced naturally in the body from the amino acids arginine, glycine and methionine. Endogenous creatine synthesis provides about half of the daily need. The remaining amount of creatine needed to maintain normal tissue levels of creatine is obtained in the diet from animal based products such as fish or meat or from dietary supplements. Creatine plays a vital role in the energy metabolism in every cell of the body. It mainly serves as a metabolic intermediary of energy transfer by facilitating the recycling of ATP, the source of energy for use and storage at the cellular level. Thus, creatine is found in high concentrations in organs with high energy turnover, with -95% of the human body's creatine stores in the skeletal muscle and the remaining 5% in the brain, liver, kidney, and testes (McCall, W., Persky, AM. Pharmacokinetics of creatine. Subcell Biochem 2007, 46, 261-273; Bonilla, D.A. et al., Metabolic Basis of Creatine in Health and Disease: A Bioinformatics-Assisted Review. Nutrients 2021, 13, 1238; Brosnan, M.E. et al., The role of dietary creatine. Amino Acids 2016, 48, 1785-1791; Harris, R. Creatine in health, medicine and sport: An introduction to a meeting held at Downing College, University of Cambridge, July 2010, Amino Acids 2011, 40, 1267; Harris, R.C.et al. Elevation of creatine in resting and exercised muscle of normal subjects by creatine supplementation. Clin. Sci. 1992, 83, 367-374; Kreider, R.B.; Stout, J.R. Creatine in Health and Disease. Nutrients 2021, 13, 447; Ostojic, S.M.; Forbes, S.C. Perspective: Creatine, a Conditionally Essential Nutrient: Building the Case. Adv. Nutr. 2021, 00, 1-4).

The administration of creatine has therefore been considered as a supportive measure for the treatment of a variety of diseases. In particular Ostojic, S.M. et al. for example describes a dietary treatment of chronical fatigue syndrome (CFS) comprising guanidinoacetic acid (GAA) (Nutrients 2016, 8, 72). The potency of creatine in the treatment of post-viral fatigue syndrome (PVFS) is discussed in Ostojic, S.M. Nutrients 2021, 13, 503; Ostojic, S.M., Therapeutic Advances in Respiratory Disease, 2020, Vol. 14, 1-2 and in Kreider R.B. et al., Nutrients 2021, 13, 447.

Starting from this the problem to be solved by the present invention is to improve the recovery from breathing difficulties (dyspnea) and/or chest pain, lung pain, and body ache caused by virus infections, particularly when these conditions are symptoms of post viral fatigue syndrome (PVFS).

### Description of the invention:

The problem is solved by administration of creatine in patients in need thereof. Creatine supports the recovery from breathing difficulties (e.g. dyspnea) and/or chest pain, lung pain, and body ache caused by virus infections.

The administration of creatine is particularly useful for patients suffering from breathing difficulties and/or chest or lung pain in combination with breathing exercises. By combination of pulmonary rehabilitation measures, such as training of the respiratory musculature by breathing exercises, and simultaneous administration of creatine, the recovery of patients suffering under breathing difficulties or chest or lung pain is significantly improved, compared to the ingestion of creatine or the application of breathing exercises alone.

Therefore, a first embodiment of the invention is a pharmaceutical composition comprising creatine or physiologically acceptable derivatives and/or salts and or adducts thereof for use in the treatment of breathing difficulties, breathlessness (dyspnea), chest / lung pain, and body ache after virus infection.

In a preferred embodiment of the invention the pharmaceutical composition comprising creatine or physiologically acceptable derivative and/or salt and or adduct thereof is used for the treatment of breathing difficulties, breathlessness (dyspnea) and/or chest (lung) pain in combination with breathing exercises.

A second embodiment of the invention is the use of creatine or physiologically acceptable derivatives and/or salts and or adducts thereof as dietary supplement or as a supplement for preparation of a diet supporting the recovery from breathing difficulties, breathlessness (dyspnea), or chest / lung pain and body ache after a viral infection of the lungs or lower respiratory system, preferably in combination with breathing exercises.

The pharmaceutical composition of the first embodiment and the composition used as dietary supplement of the second embodiment are referred to herein after also as creatine composition, creatine comprising composition or composition comprising creatine. The term "creatine composition", creatine comprising composition" or "composition comprising creatine" comprise also physiologically acceptable creatine derivatives, creatine salts and/or creatine adducts if not expressly stated otherwise.

The creatine comprising compositions of the present invention are particularly useful to support recovery from typical symptoms after virus infection, such as breathing difficulties and chest pain. Breathing difficulties or dyspnea are characterized by shortness of breath, and impaired ability to inhale or exhale. Breathing difficulties can happen as a consequence of several acute and chronic cardiopulmonary conditions, including asthma, chronic obstructive pulmonary disease, heart diseases, pneumonia etc. However, breathlessness is one of the most occurring condition after virus infection, in particular after corona virus infections including infections with SARS-CoV-2. Breathlessness or breathing difficulties often persists for weeks or months in patients with an overcome virus infection. Breathing difficulties can be further associated with low blood oxygen levels that could observed over longer periods of time and compromise general health.

Chest pain (lung pain) is a sharp throbing sensation that happens during breathing, coughing or sneezing. The most common cases of chest pain or lung pain are asthma, chronic obstructive pulmonary disease, and in particular bacterial or viral infections of pleura (pleuritis) or other thoracal tissues. Chest (lung) pain is for example observed as persistent condition after virus infection such as SARS-CoV-2 infection. Chest (lung) pain could be long lasting or chronic, and is often accompanied by cough, breathing difficulties, and wheezing. When pleuritic inflammation occurs near the diaphragm, pain can be referred also to the neck or shoulder.

Several acute and chronic viral infections which cause breathing difficulties and/or chest (lung) pain, include influenza A or B viruses (e.g., H1N1, H5N1), enteroviruses, respiratory syncytial virus, parainfluenza, adenovirus, and coronaviruses (e.g. SARS, MERS, SARS-CoV-2). The conditions can persist for weeks or months. After a SARS-CoV-2 infection, for example, breathlessness can persist from 2 weeks up to 1 year or even longer.

Breathing difficulties within the meaning of the invention comprises particularly breathlessness (dyspnea) caused by virus infection. Chest pain within the meaning of the invention includes particularly pleuritic and thoracal chest pain (lung pain) caused by viral infection.

The recovery of patients suffering under breathing difficulties or chest (lung) pain after a viral infection can be surprisingly improved by administration of creatine, in particular in combination with breathing exercises. Creatine is also known as methylguanidinoacetic acid, which is naturally occurring in the body of animals and humans. Other names for creatine are *N*-(Aminoiminiomethyl)-*N*-methyl-glycine or *N-*Methyl-*N*-guanylglycine. Creatine is further available from animal-based foods or in higher amounts as food supplement. In food supplements preferably creatine monohydrate is used, which can be prepared in very high purity.

Beside creatine, also physiologically acceptable creatine derivatives can be used in accordance with the invention. Such creatine derivatives can be natural occurring compounds, such as creatine phosphate, or pro-drugs of creatine, which are able to release creatine under physiological conditions, such as creatine esters. Physiologically acceptable creatine derivatives are preferably selected from the group consisting of creatine, creatine hydrates, creatine esters or amides, including creatine C₁-C₅-alkyl esters, N-C₁-C₅-alkyl amides, creatine phosphate, creatinol-O-phosphate or a mixture thereof.

Suitable creatine salts, creatine adducts, salts of physiologically acceptable creatine derivatives and adducts of the physiologically acceptable creatine derivatives are preferably selected from the group consisting of the corresponding acetates, citrates, maleates, fumarates, tartrates, malates, pyruvates, ascorbates, succinates, aspartates, lactates, oxalates, formates, benzoates, phosphates, sulfates, chlorides, hydrochlorides, of the corresponding potassium, sodium, calcium, magnesium salts, of the corresponding L-carnitine, acetyl-L-carnitine, taurine, betaine, choline, methionine adducts or a mixture thereof.

Pulmonary rehabilitation is a component of the management of people with respiratory diseases or lung problems due to other conditions. Pulmonary rehabilitation includes exercise training, health education, and breathing techniques aimed to improve decreased pulmonary function and improve symptoms of dyspnea. Breathing exercises are recommended during the acute management of various pulmonary diseases, including COVID-19 (Wang, T.J. et al., PM&R and Pulmonary Rehabilitation for COVID-19, accepted Article to be published in Am J Phys Med Rehab, https://www.ncbi.nlm.nih.gov/pmc/articles/PMC7315835/).

Pulmonary rehabilitation includes training of respiratory muscles and of muscles of exhalation by breathing exercises. Respiratory muscles comprise inspiratory muscles such as the diaphragm and the external intercostal muscles, e.g. musculi intercostales externi and musculi intercartilaginei, which are attached between the ribs. Diaphragm and external intercostal muscles are one of the most important groups of respiratory muscles. Further inspiratory muscles are summarized as accessory muscles of inspiration. This group of muscles support the inspiration through the lungs and comprise the, musculus serratus posterior superior, musculus serratus posterior inferior, musculus pectoralis minor et major, musculus sternocleidomastoideus, and musculus erector spinae.

The muscles of exhalation comprises internal intercostal muscles (musculi intercostales interni et intimi), lower rip muscles (musculi subcostales) and accessory exhalation muscles, such as musculus obliquus internus abdominis, musculus transversus abdominis, musculus transversus thoracis, musculus latissimus dorsi, musculus qudratus lumborum, and musculus rectus abdominis.

Treatment with creatine can already begin during the virus infection, preferably within about three months (12 weeks) after infection. Furthermore, the creatine administration should start most preferably within 4 weeks before pulmonary rehabilitation begins or concurrently with pulmonary rehabilitation. However, administration of creatine after the pulmonary rehabilitation has started is also possible.

Pulmonary rehabilitation measures, such as training of the respiratory musculature by breathing exercises should be started as soon as possible, provided that the patient's state of health permits this. The rehabilitation is usually started within 20 weeks, preferably within 12 weeks, most preferably within 6 weeks, after infection has subsided.

The duration of supplementation of creatine lasts normally between 1 week and 12 months or longer, preferably between 1 and 8 months, in particular between 3 and 6 months depending on the symptoms of the subject in need thereof.

The amount of creatine to be administered is in the range of 3g to 30g per day. Preferably the dosage is in the range of 7g to 25g, most preferred between 8g and 20g, which is higher than commonly recommended for sportsmen or sportswomen.

Preferably the administration of creatine is divided in an accumulation phase and a maintaining phase, wherein the daily dose of creatine in the composition is in the range of 10g to 30g in the initial accumulation phase and in the range of 7g to 15g for the subsequent maintaining phase. The accumulation phase has a duration of up to 3 weeks, preferably between 3 and 14 days, particularly between 5 and 10 days and the maintaining phase has a duration of 1 week to 12 months, preferably between 2 and 8 months, in particular between 3 and 6 months.

The accumulation phase is usually the initial phase. However, additional accumulation phases, for example with a duration between 1 day and 7 days, may be integrated into the maintaining phase.

The daily creatine dose can be administered once a day, during breakfast for example, or spread over 2, 3, 4 or 5 times a day.

If the administered creatine composition comprises a creatine derivative, pro drug, adduct or salt, the amount of the creatine moiety contained therein is decisive for the daily dosage within the ranges described above.

The creatine composition described herein may be preferably administered orally, in form of tablets, coated tablets, capsules, granules or powders.

In particular, granulates or powders comprising the creatine composition are used as aqueous suspension or in water-soluble form. The solubility of pure creatine and several creatine derivatives is low. Creatine for example has a solubility of 17 g/L (at 20°C). Creatine and derivatives thereof with low solubility can be used in form of an aqueous suspension. A disadvantage of an aqueous suspension is often, that it segregates before ingestion. Therefore water-soluble granules or powders are usually preferred. To increase the water solubility of creatine or creatine derivatives water soluble salts or adducts thereof may be used. To increase the water-solubility particularly the use of acids or complexing agents may be useful to provide the corresponding creatine salts or creatine derivative salts. Examples for suitable acids, including carboxylic acids, and complexing agents are selected from the group malic acid, aspartic acid, ascorbic acid, succinic acid, pyruvic acid, fumaric acid, gluconic acid, alpha-ketoglutaric acid, oxalic acid, acetic acid, formic acid, sulfuric acid, hydrochloric acid, L-carnitine, acetyl-L-carnitine, taurine, betaine, choline, and lipoic acid. Peptides and amino acids may be also useful to increase the solubility of creatine and creatine derivatives. Further sodium, potassium, calcium and magnesium salts may be used to increase the water-solubility of creatine or creatine derivatives.

The molar ratio of creatine or the creatine derivatives and the acids or complexing agents mentioned above is usually in the range of 5:1 to 1:5, preferably in the range of 2:1 to 1:2, in particular in the range of 1.3:1 to 1:1.3.

The granules and powders can also be used for preparing foodstuff supporting the recovery from breathing difficulties, breathlessness, chest / lung pain and body ache after a viral infection.

The creatine composition used in accordance with the present invention may be applied further in tablet form.

The creatine composition may be tableted as such or in form of a formulation comprising excipients. Suitable excipients are for example pharmacologically inactive ingredients, such as binders, fillers, antioxidants, preservatives, stabilizers, anti-caking agents, lubricants, disintegrants, flavors, pigments etc.

As binder or filler a wide variety of compounds may be used. Dibasic calcium phosphate; saccharides, including lactose and sucrose; polysaccharides and derivatives thereof, including starches, cellulose, modified cellulose, and cellulose ethers, such as hydroxypropyl cellulose or hydroxyethyl cellulose; microcrystalline cellulose; sugar alcohols, such as xylitol, sorbitol or mannitol; peptides, such as gelatin; polymers, e.g. polyvinylpyrrolidone, polyethylene glycol, etc. are common binders or fillers for tablets.

Typical suitable preservatives are for example cysteine, methionine, citric acid, sodium citrate, tatrazines or synthetic preservatives like parabens, including methyl paraben and propyl paraben, and benzoic acid. Suitable antioxidants may be selected from group of vitamin A, vitamin C, vitamin E, retinyl palmitate, and selenium.

Lubricants and anti-caking agents reduce the adhesion in granulates or powders and thus prevent sticking to tablet punches. They are also used to help protect tablets from sticking. The most commonly used anti-caking agents is magnesium stearate, stearic acid, or stearin, but also other magnesium or calcium salts of fatty acids may be used instead or in addition. Common mineral lubricants for example are talc or silica.

Disintegrants expand and dissolve when wet causing a tablet to break apart in the digestive tract, or in specific segments of the digestion process, releasing the ingredients for absorption by the body. Examples of disintegrants include crosslinked polymers like crosslinked polyvinylpyrrolidone (crospovidone) and crosslinked sodium carboxymethyl cellulose (croscarmellose sodium). Other suitable disintegrants are for example modified starch or sodium starch glycolate.

Flavors can be used to mask unpleasant tasting tablet ingredients. Further the ingredients may increase the patients' acceptance of the tablets. Flavorings may be natural, e.g. fruit extracts, or artificial. Natural extracts of vanilla, peach, apricot, raspberry, mint, anise or cherry can be used as flavors, for example. Antacid compounds or cough syrup would be also suitable.

Suitable pigments and colours are for example food colourants.

Tablet coatings protect tablet ingredients from deterioration by moisture in the air and make large or unpleasant-tasting tablets easier to swallow. For most coated tablets, a cellulose ether, particularly hydroxypropyl methylcellulose (HPMC) film coating is used. But other coating materials are also useful, for example synthetic polymers, shellac, plant fibers, waxes, fatty acids or polysaccharides. Capsules are coated usually with gelatine.

Particularly useful coatings for tablets related to the invention disclosed herein are crosslinked sodium carboxymethyl cellulose (croscarmellose sodium), silicified microcrystalline cellulose, agglomerated anhydrous lactose and lactitol monohydrate.

Preferred tablets comprise at least
a) 10 wt.-% to 100 wt.-%, preferably 30 wt.-% to 99 wt.-% creatine, creatine derivatives or salts or adducts thereof;
b) 0 to 80 wt.-% carbohydrates;
c) 0 to 20 wt.-% anti-caking agents selected from fatty acids or fatty acid salts, particularly magnesium stearate;
d) 0 to 20 wt.-% acidifier, preferably citric acid;
e) 0 to 20 wt.-% fatty acids, preferably coconut oil;
f) 0 to 5 wt.-% flavors.

Preferred capsules comprise at least
a) 10 wt.-% to 95 wt.-%, preferably 30 wt.-% to 80 wt.-% creatine, creatine derivatives or salts or adducts thereof
b) 0 to 80 wt.-% carbohydrates;
c) 0 to 20 wt.-% anti-caking agents selected from fatty acids or fatty acid salts, particularly magnesium stearate,
d) 0 to 5 wt.-% flavors;
e) 5 to 90 wt.-% gelatin.

The described ingredients may be useful not only in tablets but also in granules or powders comprising creatine or creatine derivatives or salts or adducts thereof. Particularly binders, fillers, antioxidants, preservatives, stabilizers, anti-caking agents, lubricants, disintegrants, flavors, and pigments can be part of the creatine composition used in accordance with the present invention.

Preferred granules or powders relating to the described invention comprise 10 wt.-% to 100 wt.-%, preferably 30 wt.-% to 99 wt.-% creatine, creatine derivatives or salts or adducts thereof.

In combination with creatine, creatine derivatives or salts or adducts thereof, anti-inflammatory drugs can be applied to support the recovery from breathing difficulties (dyspnea) and/or chest pain, lung pain, and body ache caused by virus infections. Anti-inflammatory drugs are for example non-steroidal drugs, such as aspirin, ibuprofen, naproxen, diclofenac, celecoxib, mefenamic acid, etoricoxib, indomethacin etc. or steroids, such as corticosteroids, including cortisone, hydrocortisone and prednisone.

Further combinations of creatine, creatine derivatives or salts or adducts thereof, with neuroprotective drugs can be advantageous. Preferred neuroprotective agents include glutamate excitotoxicity inhibitors such as ginsenoside, riluzole, progesterone, estrogen, memantine, or simvastatin; stimulants such as caffeine; growth factors such as IGF-1, CNTF; nitric oxide synthase inhibitors; and caspase inhibitors or erythropoietin.

A combination of creatine, creatine derivatives or salts or adducts thereof, with drugs for the treatment of the virus infection is also possible, in particular if the administration of creatine is started during the acute phase of the virus infection.

For the treatment of COVID-19 drugs of the group Lagevrio (molnupiravir), Olumiant (baricitinib), (tixagevimab / cilgavimab), Kineret (anakinra), Paxlovid (PF-07321332 / ritonavir), Regkirona (regdanvimab), RoActemra (tocilizumab), Ronapreve (casirivimab / imdevimab), Veklury (remdesivir), Xevudy (sotrovimab) are available for example. For the treatment of influenza Rapivab (peramivir), Relenza (zanamivir), Tamiflu (oseltamivir phosphate), Xofluza (baloxavir marboxil) are suitable drugs. For the treatment of adenoviruses cidofovir, ribavirin, ganciclovir, and vidarabine, are useful drugs.

Creatine, creatine derivatives or salts or adducts thereof are preferably used in combination with a pain-reducing, anti-inflammatory diet according to a preferred embodiment of the present invention. The preferred diet shall be provide all nutrients for normal energy metabolism and healthy function of the nervous system. A particularly suitable diet should comprise vitamins, minerals, unsaturated fatty acids, amino acids, secondary plant metabolites, including anti-oxidative agents, phytonutrients or essential or semi-essential nutrients.

For a pain-reducing, anti-inflammatory diet particularly vitamins of the group of vitamin, C, vitamin D, vitamin E, vitamin K and B vitamins (thiamine, riboflavin, nicotinamide, pantothenic acid, pyridoxine, biotin, folate, and/or vitamin B₁₂) should applied in sufficient amounts.

Important minerals are selected from group of magnesium, calcium, potassium, sodium, copper, manganese, zinc, selenium, and boric acid / boron.

The vitamins and minerals should be present in the diet in sufficient quantities so that no deficiency symptoms occur. Appropriate recommendations for the daily amount of these nutrients are issued by the German Society for Nutrition (Deutsche Gesellschaft für Ernahrung e.V., Referenzwerte für die Nährstoffzufuhr, 2. Auflage, 7. Aktualisierte Ausgabe, 2021).

A further group of useful nutrients for a pain-reducing, anti-inflammatory diet are unsaturated fatty acids, in particular omega-3 fatty acid; docosahexanoic acid (DHA), eicosapentenoic acid (EPA), alpha-lipoic acid and lecithine.

A preferred diet should be also rich on amino acids selected from the group of L-tyrosine, arginine and glycine. Additional amino acids for example theanine, cystine, taurine, or mixtures thereof are enriched in a preferred diet.

Further compounds that should be present in a pain-reducing, anti-inflammatory diet are N-acetyl-L-cysteine, gamma-amino-butyric acid (GABA), S-adenosylmethionine (SAMe), ubiquintol (coenzyme Q10), NADH, resveratrol, lutein, lycopene, choline, and carnitine.

Phytonutrients, including secondary plant metabolites, which are particularly useful in a pain-reducing, anti-inflammatory diet are for example antioxidants, anthocyanidines, flavonoids, flavones, isoflavones, catechins, anthocyanidins, isothiocyanates, carotenoids, allyl sulfides, polyphenols, resveratrol, lutein, or lycopene. Particularly preferred secondary plant metabolites are oligomeric procyanidins and oligomeric proanthocyanidins (OPCs).

For a preferred diet also plants, particularly herbs, spices, fruits, vegetables and legumes, or extracts, oils, powders, or compounds thereof can be used, for example from the group boswellia serrata, curcuma longa, grape seeds (in particular with OPCs), or devil's claw. Further tomatoes, olive oil, green leafy vegetables, such as spinach, kale, and collards, nuts like almonds and walnuts, or fruits such as strawberries, blueberries, cherries, and oranges are regarded as anti-inflammatory foods. Fatty fish like salmon, mackerel, tuna, and sardines have also anti-inflammatory effects.

The recommended nutrients for a pain-reducing, anti-inflammatory diet can be supplied by selecting suitable foods or by addition of the respective components via nutrition supplements.

### Description of the figures:

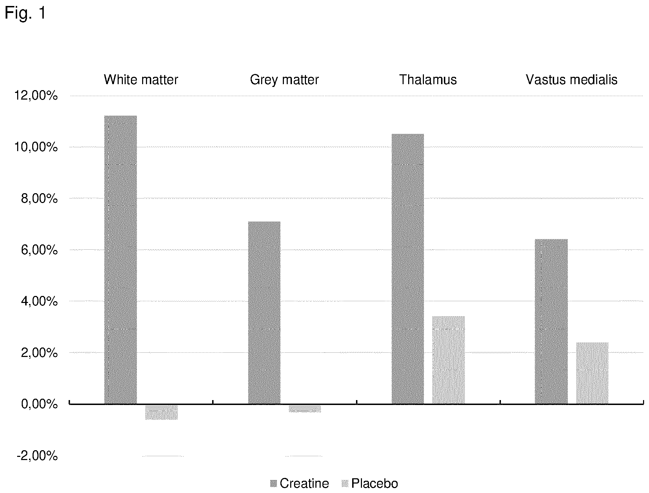

Figure 1: Changes in tissue creatine levels in patients suffering under Post-COVID. Left columns representing the group of patients receiving creatine, right columns representing the placebo group.

### Working examples:

A study was conducted to evaluate effectiveness and safety of replenishing creatine via dietary supplementation in patients suffering under Post-COVID after a SARS-CoV-2 infection. This study employed a parallel-group, randomized placebo-controlled double-blind design. The allocation ratio to experimental group (creatine) and control group (placebo) was set at 1:1. The eligibility criteria for patients to be included in the trial were: age 18-65 years, COVID-19 positive test within last 3 months (as documented by valid PCR or antigen test) and at least one of additional COVID-19 related symptoms selected from the group of anosmia, ageusia, breathing difficulties, lung pain, body aches, headaches, and concentration difficulties.

Exclusion criteria were other pulmonary and cardiovascular conditions, and history of dietary supplement use during the 4 weeks before the trial commences.

The study was conducted in compliance with the Declaration of Helsinki (7th revision). The data presented until now were collected at FSPE Applied Bioenergetics Lab at the University of Novi Sad, from October 2021 to January 2022. The experimental (creatine) group received 4 grams of creatine monohydrate per day, while control group (placebo) received equivalent amount of inulin. The participants were asked to take the intervention one time per day during breakfast, with experimental or control powder stirred in 250 mL of lukewarm water and consumed immediately afterwards. Both interventions were similar in appearance, texture, and sensory characteristics. Creatine monohydrate was provided by Alzchem Trostberg GmbH (Trostberg, Germany). The duration of intervention so far was 3 months, and participants were asked to refrain from using any other dietary supplements during the trial. All outcome measures were determined at baseline (pre-administration) and after 3 months. The primary outcome was the change in vastus medialis creatine levels and the creatine levels in brain at baseline and at 3-month follow-up. The minimal sample size (n = 12) was calculated using power analysis (G*Power 3.1.9.3, Heinrich-Heine-Universitat Düsseldorf), with the effects size set at 0.50 (medium effect), alpha error probability 0.05, power 0.80 for two groups, and two measurements of study outcomes.

The number of participants who were randomly assigned, received intended treatment, and were analyzed for the primary outcome at this time was twelve in total, six in experimental group and six in control group. The participants recruited reported no major side effects of either intervention so far.

Accumulation of creatine in skeleton muscles in Post-COVID patients.

Tissue levels of creatine were measured with proton magnetic resonance spectroscopy (1.5 T Avanto scanner, Siemens, Erlangen, Germany) using matrix head coil in circularly polarized mode, with metabolite spectra in the specific regions of skeletal muscle and brain (vastus medials muscle, thalamus, frontal, precentral, paracentral, and parietal white and grey matter) processed as previously described (Appl Physiol Nutr Metab. 2016 Sep, 41(9):1005-7.).

Changes in tissue creatine levels in patients suffering under Post-COVID are summarized in Table 1. The Table shows changes in tissue creatine levels in white matter (brain), thalamus, vastus medialis and grey matter (brain) after intervention with 4 gram creatine per day for 3 months compared to creatine level of the placebo group.

**Table. 1. Changes in tissue creatine level (mM)**

| | Creatine | | Placebo | |
|---|---|---|---|---|
| Total creatine (mM) | Baseline | 3 months | Baseline | 3 months |
| Vastus medialis muscle | 28.2 ± 3.0 | 30.0 ± 4.2 | 20.6 ± 1.9 | 21.1 ± 3.1 |
| Thalamus | 5.7 ± 0.5 | 6.3 ± 0.9 | 5.9 ± 1.5 | 6.1 ± 0.8 |
| Left frontal white matter | 5.9 ± 0.6 | 6.2 ± 0.6 | 5.8 ± 0.6 | 5.7 ± 0.6 |
| Right frontal white matter | 5.6 ± 0.8 | 6.6 ± 0.8 | 5.7 ± 0.8 | 5.6 ± 0.7 |
| Left frontal grey matter | 6.6 ± 0.4 | 6.9 ± 0.7 | 6.2 ± 0.7 | 6.2 ± 1.1 |
| Right frontal grey matter | 6.5 ± 0.7 | 6.2 ± 0.3 | 6.4 ± 0.7 | 6.3 ± 0.7 |
| Left precentral white matter | 6.0 ± 0.6 | 6.2 ± 0.8 | 5.8 ± 0.4 | 5.8 ± 0.3 |
| Right precentral white matter | 5.8 ± 0.5 | 5.8 ± 0.7 | 5.8 ± 0.3 | 5.8 ± 0.3 |
| Left precentral grey matter | 6.7 ± 0.6 | 7.3 ± 0.9 | 6.5 ± 0.4 | 6.3 ± 0.6 |
| Right precentral grey matter | 6.8 ± 0.4 | 7.2 ± 0.5 | 6.7 ± 0.4 | 6.8 ± 0.4 |
| Left parietal white matter | 5.3 ± 0.6 | 6.0 ± 0.9 | 5.2 ± 0.4 | 5.1 ± 0.4 |
| Right parietal white matter | 5.3 ± 0.7 | 6.9 ± 0.9 | 5.1 ± 0.6 | 5.2 ± 0.8 |
| Left mesial grey matter | 7.2 ± 0.9 | 8.0 ± 1.2 | 7.0 ± 0.7 | 7.0 ± 0.8 |
| Right mesial grey matter | 7.1 ± 0.3 | 8.2 ± 1.3 | 7.1 ± 0.4 | 7.2 ± 0.5 |

The differences in percent of the creatine level after three months and the corresponding baseline level in white matter (brain), grey matter (brain), thalamus, and vastus medialis muscle calculated from Table 1 are shown in Figure 1. Left columns representing the group of patients receiving creatine, right columns representing the placebo group. For the values in white matter and grey matter average values are determined from Table 1.

Effect of creatine as nutrition supplement for Post-COVID patients.

The patient-reported outcomes for COVID-19-related signs and symptoms (e.g., breathing difficulties, lung pain, body aches,) were evaluated with VAS scale.

**Table. 2**

| | Creatine | | Placebo | |
|---|---|---|---|---|
| | Baseline | 3 months | Baseline | 3 months |
| Breathing difficulties | 2.3 | 0.5 | 2.9 | 1.3 |
| Lung pain | 2.3 | 0.5 | 2.0 | 0.2 |
| Body Aches | 4.5 | 1.2 | 4.2 | 1.3 |

Breathing difficulties (dyspnea) are significantly reduced for 78% after creatine intake as compared to 55% after placebo intake. Body aches is reduced slightly for 73.3% after creatine intake as compared to 69% after placebo intake. Lung pain is reduced significantly in the creatine cohort.

In this stage of the randomized controlled trial, it was found that creatine is accumulated in skeletal muscle and brain when administered for 3 months. An increase in time to exhaustion in creatine group, with creatine outcompetes placebo in prolonging time to exhaustion after 3-month administration of creatine. The improved endurance is associated with a reduction in body aches and lung pain and in particular with the recovery from breathing difficulties. In addition, creatine induced no major side effects.

Combination of creatine supplementation and pulmonary rehabilitation:
Pulmonary rehabilitation is performed in accordance with Wang T.J. et al., Am J Phys Med Rehabil, 2020, Jun 11 (DOI 10.1097/PHM.0000000000001505 / PMCID: 7315835). Pulmonary rehabilitation is tailored to each individual patient and includes for example modified segmental breathing, breathing exercises for strengthening respiratory muscles and muscles of exhalation, inspiratory muscle training, bed mobility exercise, stretching, gymnastics, and/or walking. The extent of the exercises is low at the beginning and is slowly increased without exposing the patient higher efforts.

The exercise frequency is 2 to 4 times per day for 10 to 15 minutes. The exercise time can be increased slowly to up to 30 minutes.

The administration of creatine in combination with pulmonary rehabilitation (breathing exercises has a synergistic effect on the recovery of subjects suffering under Post-COVID syndrome with breathing difficulties or chest (lung) pain or both.

## Claims

1. Pharmaceutical composition comprising creatine or physiologically acceptable derivatives and/or salts and or adducts thereof for use in the treatment of one of the conditions selected from group of breathing difficulties, chest pain, lung pain, and body ache, after virus infection.

2. The pharmaceutical composition of claim 1, wherein the breathing difficulties or lung pain are caused by a coronavirus infection or are symptoms of Post-COVID.

3. The pharmaceutical composition of claim 1 or 2 for use in the treatment of breathing difficulties, chest pain or lung pain in combination with breathing exercises.

4. The pharmaceutical composition of claim 1 or 2, wherein the composition is for use in combination with breathing exercises for strengthening respiratory muscles including strengthening of the diaphragm, the external intercostal muscles and the muscles of exhalation.

5. The pharmaceutical composition of any one of claims 1 to 3, wherein the composition is for use in combination with breathing exercises and wherein the breathing exercises are gradually increased as tolerated by the subject in need thereof.

6. The pharmaceutical composition of any one of claims 1 to 4, wherein the physiologically acceptable creatine derivative is selected from the group consisting of creatine, creatine hydrates, creatine esters or amides, including creatine C₁-C₅-alkyl esters, N-C₁-C₅-alkyl amides, creatinol, creatinol-O-phosphate or a mixture thereof.

7. The pharmaceutical composition of any one of claims 1 to 5, wherein the creatine salts and adducts or the salts and adducts of the physiologically acceptable creatine derivatives are selected from the group consisting of the corresponding acetates, citrates, maleates, fumarates, tartrates, malates, pyruvates,
ascorbates, succinates, aspartates, lactates, oxalates, formates, benzoates, phosphates, sulfates, chlorides, hydrochlorides, of the corresponding potassium, sodium, calcium, magnesium salts, of the corresponding L-carnitine, acetyl-L-carnitine, taurine, betaine, choline, methionine adducts or a mixture thereof.

8. The pharmaceutical composition of any one of claims 1 to 6, wherein the composition comprises creatine monohydrate.

9. The pharmaceutical composition of any one of claims 1 to 7, wherein the daily dose of creatine in the composition is in the range of 7g to 30g.

10. The pharmaceutical composition of any one of claims 1 to 8, wherein the daily dose of creatine in the composition is an accumulation dose in the range of 10g to 30g for an initial accumulation phase and a maintaining dose in the range of 7g to 15g for a subsequent maintaining phase, wherein the accumulation phase has a duration of up to 3 weeks and the maintaining phase has a duration of 1 month to 12 months.

11. Use of a composition comprising creatine or physiologically acceptable derivatives and/or salts and or adducts thereof as dietary supplement or as a supplement for preparation of a diet supporting the recovery from one of the conditions selected from group of breathing difficulties, chest pain, lung pain, and body ache after viral infection.

12. The use of claim 11, wherein the breathing difficulties or lung pain are caused by a coronavirus infection of the lungs or lower respiratory system or are symptoms of Post-COVID.

13. The use of claim 11 or 12, wherein the composition is used in combination with breathing exercises for strengthening respiratory muscles including strengthening of the diaphragm, the external intercostal muscles and the muscles of exhalation.

14. The use of any one of claims 11 to 13, wherein the daily dose of creatine in the composition is in the range of 7g to 30g.

15. The use of any one of claims 11 to 14, wherein the daily dose of creatine in the composition is an accumulation dose in the range of 10g to 30g for an initial accumulation phase and a maintaining dose in the range of 7g to 15g for a subsequent maintaining phase, wherein the accumulation phase has a duration of up to 3 weeks and the maintaining phase has a duration of 1 month to 12 months.

16. The use of any one of claims 11 to 15, wherein the used composition comprises creatine monohydrate.
